# EUROPEAN PATENT SPECIFICATION

(11) **EP 4 771 466 B1**
(45) Date of publication and mention of the grant of the patent: **30.09.2026**
(21) Application number: 24821746.5
(22) Date of filing: 21.11.2024
(51) Int. Cl.: G06F 3/01, A61B 3/113, A61B 5/16, G02B 27/00, G02B 27/01

(54) **EDGE-COMPUTED EYE DESCRIPTOR UNIT CONNECTABLE TO COMPUTER APPLICATION DEVICES**
KANTENBERECHNETE AUGENDESKRIPTOREINHEIT, DIE AN COMPUTERANWENDUNGSVORRICHTUNGEN ANSCHLIESSBAR IST
UNITÉ DE DESCRIPTION OCULAIRE À CALCUL EN PÉRIPHÉRIE CONNECTABLE À DES DISPOSITIFS D'APPLICATION INFORMATIQUE

(43) Date of publication of application: 08.07.2026
(73) Proprietor: Viewpointsystem GmbH, 1010 Wien (AT)
(72) Inventor: ORTS, Javier, 1160 WIEN (AT); LINSENMAIER, Frank, 71384 Weinstadt (AT); BERGER, Nils, 9494 Schaan (AT)
(74) Representative: Schardmüller Gall-Schuhmann Patentanwälte OG
(86) International application number: PCT/EP2024/083187
(87) International publication number: WO 2026/109147

(56) References cited:
- US-A1- 2021 011 288
- US-A1- 2021 116 995
- US-A1- 2024 377 648
- ANONYMOUS: "Eye tracking", 1 October 2024 (2024-10-01), Wikipedia, XP093275193, Retrieved from the Internet <URL:https://en.wikipedia.org/w/index.php?title=Eye_tracking&oldid=1248764651> [retrieved on 20250507]
- ANONYMOUS: "Neural processing unit", 29 October 2024 (2024-10-29), Wikipedia, XP093275187, Retrieved from the Internet <URL:https://en.wikipedia.org/w/index.php?title=Neural_processing_unit&oldid=1254068429> [retrieved on 20250507]
- ANONYMOUS: "Bare machine", 22 June 2024 (2024-06-22), Wikipedia, XP093275197, Retrieved from the Internet <URL:https://en.wikipedia.org/w/index.php?title=Bare_machine&oldid=1230469800> [retrieved on 20250507]
- ANONYMOUS: "Real-time operating system", 27 October 2024 (2024-10-27), Wikipedia, XP093275201, Retrieved from the Internet <URL:https://en.wikipedia.org/w/index.php?title=Real-time_operating_system&oldid=1253656346> [retrieved on 20250507]

## Description

### Technical Field

The present invention discloses an edge-computed eye descriptor unit connectable to computer application devices, a method to process user's eye descriptors using the edge-computed eye descriptor unit and a computer application device including the edge-computed eye descriptor unit.

An eye descriptor unit, commonly known as eye/gaze-tracking device, within the scope of this document, refers to a specialized electronic system designed to detect, measure, and analyze ocular movements with a certain degree of accuracy. These devices, typically comprising a combination of eye sensors, for example imaging cameras, and software algorithms, monitor the position, movement, and gaze direction of the user's eyes. By capturing data on where a user is looking, an eye-tracking device provides insights into attention, interest, and visual engagement, which may be applied across various fields, including assistive technologies, behavioral research, user interface optimization, and immersive virtual reality systems.

Due to their ability to non-invasively and accurately interpret human visual attention, eye-tracking devices are increasingly integrated into technologies for medical diagnostics, gaming, advertising analytics, and human-computer interaction.

Head-mounted eye-tracking technology refers to a class of wearable systems specifically designed to track eye movements from a device affixed directly to the user's head (so-called head-mounted device). These devices are generally more accurate in dynamic environments, as they move in concert with the user's head, maintaining a consistent viewpoint for tracking ocular activity. Head-mounted eye/gaze trackers offer a range of designs, each leveraging different configurations of sensors, cameras, and optical components to suit varied application requirements.

Are known wearable display-integrated eye-trackers, which are built into head-mounted displays (HMDs), such as virtual reality (VR) or augmented reality (AR) headsets. These systems employ small cameras or optical sensors embedded within the display near the lenses, allowing for real-time gaze tracking alongside immersive visual experiences. They are integral in VR and AR applications where the user's gaze must be continuously tracked to enhance interactive elements, improve rendering techniques (such as foveated rendering), or study user behavior within a virtual environment.

Smart glasses with embedded eye-tracking technology represent a subset of head-mounted devices where eye-tracking components are integrated into eyewear that resembles traditional glasses. These systems are typically lightweight and designed for extended wear, making them ideal for applications requiring unobtrusive, long-duration tracking, such as behavioral research in naturalistic settings or real-world advertising studies. Smart glasses utilize miniature cameras or sensors, usually embedded within the frame, to track eye movement with minimal impact on the wearer's mobility.

Head-mounted eye-tracking devices utilize a range of specialized processors to support the demanding requirements of eye data processing, encompassing real-time gaze detection, tracking, and data interpretation. The processors integrated within these devices-namely central processing units (CPUs), graphics processing units (GPUs), digital signal processors (DSPs), and application-specific integrated circuits (ASICs)-each contribute unique computational advantages tailored to the requirements of continuous, high-precision eye tracking.

Central processing units (CPUs) provide foundational control and general-purpose computing within the device, managing system operations and executing control algorithms that enable seamless interaction between various processing units. The CPU often coordinates the input from optical sensors or cameras and ensures that data is routed efficiently for further processing.

For the computationally intensive tasks of image analysis and real-time data visualization associated with eye tracking, many systems incorporate graphics processing units (GPUs). GPUs are highly suited for parallel processing, a requirement in high-resolution image capture and processing, where rapid computation is essential to identify and interpret eye features accurately. This capability is particularly beneficial in applications such as virtual and augmented reality, where immediate and precise gaze mapping enhances user experience and interactivity.

Digital signal processors (DSPs) are frequently utilized in head-mounted eye-tracking devices due to their high efficiency in handling real-time signal-processing tasks, such as filtering, feature extraction, and noise reduction. DSPs support continuous, low-latency processing with optimized power efficiency, making them ideal for wearable, battery-operated devices where extended operational life is necessary.

In scenarios that demand exceptionally high processing speed and minimal delay, particularly in specialized or medical applications, application-specific integrated circuits (ASICs) may be incorporated. ASICs are custom designed to perform eye data processing functions, delivering high accuracy through dedicated circuitry tailored to the exact computational demands of eye tracking.

By integrating CPUs for system control, GPUs for image processing, DSPs for efficient signal handling, and ASICs for specialized performance, head-mounted eye-tracking devices achieve the comprehensive processing capabilities required for accurate, real-time gaze tracking across a variety of environments and applications.

### Prior Art

The European Patent EP4281829 describes an advanced eye-tracking kit designed specifically for integration with regular eyeglasses. This invention targets the field of wearable technology, specifically enhancing user interaction and experience by monitoring eye movement and gaze direction. The design outlines a modular system that includes an eye-tracking component fitted onto or embedded within the frame of eyeglasses. The system consists of multiple elements such as sensors, processors, and communication units, optimized for minimal size to ensure comfort and usability for extended wear.

One of the key features highlighted in the patent is the emphasis on non-intrusive user experience, whereby the eye-tracking kit can be seamlessly attached to conventional eyewear without requiring extensive modifications. This adaptability aims to make eye-tracking more accessible and practical for various applications, including augmented reality (AR), virtual reality (VR), and user interface control systems.

However, EP4281829 is focused on mechanical form-factor aspects of the eye-tracking kit, not on reducing its power consumption, improving response times, or saving bandwidth for the unit itself.

Are also known system architectures designed to enhance the integration and performance of electronic systems such as AR/VR/XR headsets where such architectures are connected. Generally, these architectures include the use of a general-purpose chipset, characterized by the incorporation of multi-core processing capabilities for processing the eye data directly in the AR/VR headset. Processing power for AR applications is generally managed within the headset itself and requires external computing support for more complex tasks. Such kind of architecture is heavily power consuming, subjects to a certain degree of latency and has a negative impact on real-time eye-data processing.

From the prior art (US 2024/0377648 A1), also nose pad eye tracking modules shaped in the form of a wearable U-shaped nose bridge portion are known, applicable to augmented/virtual reality glasses. While such eye tracking modules according to the prior art may represent eye tracking units that can be connected to computer application devices when in use, they lack an edge computation unit provided with a machine learning hardware accelerator support configured to receive as input unprocessed user's eye representation data from eye sensors and send as output an array of user's eye descriptors. Further, such known eye tracking modules also lack an edge computation unit being a microcontroller programmed with a customized bare metal code or with an RTOS or with a combination of both.

From the prior art (US 2021/0011288 A1) also systems and methods for distributing a neural network across multiple computing devices in the context of a head-mounted display (HMD) for augmented or virtual reality device are disclosed. A first device, typically a CPU, GPU or local processing circuitry of the HMD, implements a first set of layers of a neural network, while a second device - a remote server, an AI accelerator or another computer with higher processing capabilities - implements the remaining layers. US 2021/0011288 A1 in particular does not disclose an eye descriptor unit shaped for being integrated into a U-shaped nose bridge portion.

### Purposes of the Invention

One objective of the present invention, according to a first of its aspects, is obtaining an eye descriptor unit, capable of executing eye/eye descriptor computations with minimal energy consumption, that can be connected to many possible computer application devices, therefore not only VR/XR headsets as computer application devices but also smartglasses like AR glasses with minimalistic Hardware structure. This approach allows the unit to support real-time eye-tracking functionalities while maintaining a low power profile, optimized computational efficiency, and reduced heating dispersion issues, thus enhancing the overall usability of the eye descriptor unit.

A second objective of the present invention is to provide an eye descriptor unit with significantly optimized latency and throughput of low-volume time-series asset data.

A third objective of the present invention is to provide an eye descriptor unit more secure, and it computes the complete eye descriptor data in the eye descriptor unit itself, reducing the unnecessary traffic to the central repository of the computer application device, which is the recipient of the user's eye descriptor data already processed.

A fourth objective of the present invention is providing an eye descriptor unit, having minimal impact, compact structure being able to be easily connected to every kind of computer application device.

A fifth objective of the present invention is to provide an eye descriptor unit that is able to not interfere with the user's nose, and/or with the user's field of view, once connected to the computer application device.

A further objective of the present invention is to provide an eye descriptor unit that is very lightweight, minimizing the total weight of the computer application device.

Another objective of the present invention is to provide a method for detecting user eye descriptors using an eye descriptor unit according to the aspects mentioned above.

Another objective of the present invention is to provide a computer application device implementing the eye descriptor unit using the method mentioned above.

### Summary of the Invention

Hereinafter are summarized some technical aspects of the present invention which enable some of the most important purposes to be achieved.

According to a first aspect, this invention relates to an eye descriptor unit for estimating the user's gaze once the eye descriptor unit is connected to a computer application device worn by a user, said eye descriptor unit comprising at least one eye sensor facing the user's left eye and one eye sensor facing the user's right eye, an edge computation unit provided with a machine learning hardware accelerator support and configured to receive as input unprocessed user's eye representation data from the eye sensors and to send as output an array of user's eye-descriptors when in use and wherein the edge computation unit is programmed with a customized bare metal code or with Real-Time Operating System RTOS for microcontrollers or with a combination of customized bare metal code and Real-Time Operating System RTOS for microcontrollers.

Such a unit is very compact and particularly low power consuming, optimized to run the entire eye/tracking pipeline directly in the edge computing unit, receiving as input unprocessed data from the eye sensors and outputting small-sized processed data files.

The edge computation unit is programmed with a customized bare metal code or with Real-Time Operating System RTOS for microcontrollers or with a combination of customized bare metal code and Real-Time Operating System RTOS for microcontrollers, entailing NO OS (Operative System) Overhead. The absence of background tasks or OS-induced wakeups means the eye descriptor unit object of the present invention consumes power only for tasks explicitly defined by the customized bare metal code or Real-Time Operating System RTOS for microcontrollers or both of the edge computing unit processing data directly from the eye sensors.

According to a second aspect, this invention relates to an eye descriptor unit wherein the edge computed unit is configured to implement a method accurate and robust enough to predict user's eye descriptors.

According to a third aspect, this invention relates to an eye descriptor unit configured to reduce further its power consumption by operating an alternate working/sleeping mode.

According to a fourth aspect, this invention relates to a method for detecting user's eye descriptors using the eye descriptor unit, once it is connected to a computer application device.

According to a fifth aspect, this invention relates to a computer application device implementing the eye descriptor unit and to a method for detecting user's eye descriptors using the eye descriptor unit, according to what is claimed in the dependent claims of the present specification

### Brief Description of Drawings

The structural and functional features of the present invention and its advantages with respect to the known prior art will become even clearer from the underlying claims, and in particular from an examination of the following description, made with reference to the attached figures which show a preferred but not limited schematic embodiment of the invented method, system, device, in which:
Figure 1 illustrates the eye descriptor unit according to the present invention;
Figure 2 illustrates the first preferred embodiment of the hardware architecture of the eye descriptor unit according to the present invention, once connected with the computer application device 2;

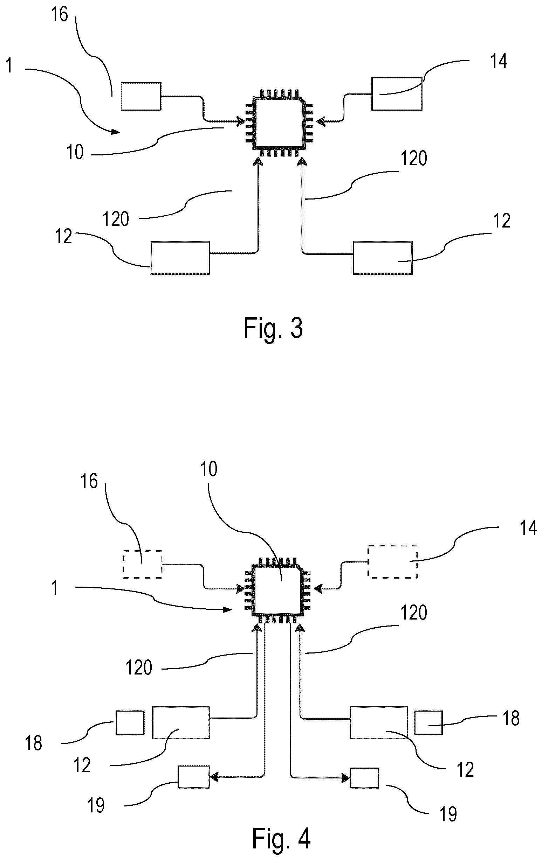

Figures 3 and 4 illustrate other preferred embodiments of the eye descriptor unit according to the present invention; and

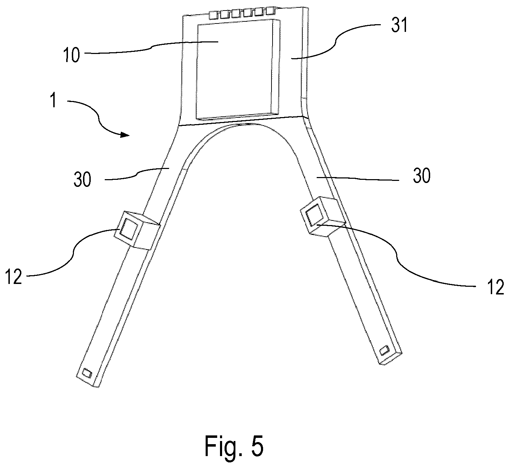

Figure 5 illustrates an embodiment of the PCB architecture of the eye descriptor unit according to the present invention.

### Modes for Carrying out the Invention

In general, this disclosure describes an eye descriptor unit connectable to a computer application device, a method for computing user's gaze vector by using the eye descriptor unit.

The specifications "right" or "left" or "high" or "low" or "front" or "back" relate to the intended manner of wearing eyeglasses and the eye tracking module by a human being.

It should highlight that for nose radix it is intended a depression at the root of the nose, which defines the nasal root and the origin of the nose from the point of the glabella. The radix extends inferiorly from the nasion to the level of a horizontal line passing through the lateral canthi, and superiorly from the nasion for an equivalent distance.

Furthermore, for nasal bones are intended two small oblong bones, varying in size and form in different individuals; they are placed side by side at the middle and upper part of the face and by their junction, form the bridge of the upper one-third of the nose.

Regarding eye-tracking glasses in general, it shall be highlighted that they have at least a part of their frame having the purpose to retaining the lenses, which may be prescription lenses or sun lenses or specific filtering/protective lenses. Furthermore, they have at least a portion, so-called nose bridge, acting as a spacer between the two lenses, able to create the space for the user's nose.

Nowadays are known VR/AR/XR headsets and so-called smartglasses. In some cases these devices have a very minimal spectacles frame, including a horizontal upper part provided with an element constituting the nose frame, in order to have the device placed stably and supported by the nose of the user.

VR headsets are designed to fully immerse users in a computer-generated environment, separating them from the physical world and creating a realistic virtual space. Their main components include high-resolution OLED or LCD display panels positioned close to the eyes, providing stereoscopic 3D images by showing slightly different visuals to each eye. To enhance this immersion, VR headsets use lenses that focus and reshape images. They also incorporate motion sensors, including accelerometers, gyroscopes, and magnetometers, to track head movements and update the display in real-time, reducing motion sickness and enhancing realism. Some systems add external tracking sensors, such as cameras placed around the environment, to achieve more precise positional tracking. Many VR headsets integrate spatial audio systems or headphones for synchronized audio, while handheld controllers with touchpads, buttons, and sensors enable user interaction. The computing power required for VR can be provided by an external computer or, in standalone devices, through onboard processors.

In contrast, AR headsets are designed to overlay digital information onto the real world, providing a mixed-reality experience that enables users to view and interact with virtual objects within their physical environment. These headsets include transparent or semi-transparent display systems, like optical waveguides or reflective displays, that blend digital imagery with the real world by projecting images through micro-projectors onto the lenses. To anchor these virtual images effectively, AR headsets use a combination of lenses and optics to ensure clear and distortion-free visuals. Advanced AR headsets may rely on Simultaneous Localization and Mapping (SLAM) sensors, including cameras and depth sensors, to scan and map the user's physical environment. This tracking allows virtual objects to remain stable relative to the real-world setting. Additionally, many AR headsets employ bone-conduction speakers or open-air audio systems that deliver sound without isolating the user from real-world audio cues. In terms of control, AR devices may incorporate gesture-tracking sensors, allowing users to interact with virtual objects through hand and finger movements. Processing power for AR applications is generally managed within the headset itself, though some devices may require external computing support for more complex tasks.

Furthermore, Extended Reality (XR) headsets represent a broad category that encompasses the full spectrum of immersive technologies, including elements of both VR and AR, and often add enhanced functionalities for a more versatile user experience. XR headsets aim to merge the immersive capabilities of VR with the interactive, real-world integration of AR, allowing users to switch seamlessly between fully virtual environments, augmented overlays, and combinations of both. These devices often may include high-resolution displays with adaptive optics that can adjust the transparency or opacity of the visuals, enabling a smooth transition from complete immersion in virtual content to partially transparent overlays on the real world. With powerful onboard processors or wireless connections to external computers, XR headsets handle complex computations needed for fluid transitions between virtual and augmented experiences, making them suitable for a wide array of applications, from professional training and simulation to entertainment and remote collaboration.

So-called Smart glasses, on the other hand, provide a more lightweight experience, offering heads-up display (HUD) capabilities for simpler applications like notifications, navigation, and hands-free interaction. Unlike VR/AR and XR headsets, smart glasses do not focus on immersion and often have lower display resolutions, using compact display modules like Micro-LEDs or monochrome OLEDs. They rely on simple optics, such as prisms or waveguides, to project basic visual information onto the user's field of vision. Because smart glasses are designed primarily for low-power, everyday use, they typically lack the environment-mapping and SLAM sensors of AR headsets. However, they may still include very basic motion sensors like accelerometers and gyroscopes to support simple tracking functions. For interaction, many smart glasses utilize voice commands or touch-sensitive controls on the frame. The onboard processor is generally optimized for energy efficiency to extend battery life, supporting low-power functionalities for daily use.

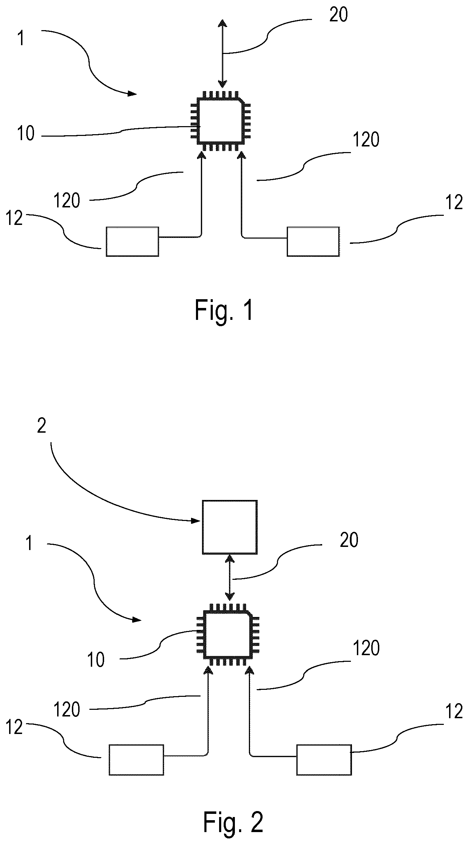

From now on, a general computer application device 2 is intended for instance VR/AR/XR headsets, so-called smart glasses or any electronic device connectable to the eye descriptor unit 1 (fig. 2).

The eye descriptor unit 1, according to an embodiment of the present invention, is a hardware structure which is shaped to be integrated into a Wearable U-shaped nose bridge portion compatible with the nose of a wearer when in use, thus being configured to make the computer application device 2 easily customizable on different nose shapes when the eye descriptor unit 1 is implemented into said computer application device 2. The above-mentioned configuration may be implemented in all the embodiments disclosed in the present specification. The nose bridge is a portion necessary in all eyeglasses, but in some cases, this portion is not clearly detectable, like for example in the ones configured like safety goggles, where a visor replaces the lenses. In some cases, like smart glasses provided with eXtended technology, the central portion of the upper frame of the smart glasses is provided with an element constituting the nose frame, in order to have the device placed stably and supported by the nose of the user. Therefore, the eye descriptor unit 1 may be integrated into such a part defined as the nose frame. Another possible mechanical configuration provides for at least the eye sensors 12 of the eye descriptor unit 1 placed in a first PCB nose bridge portion and the edge computing unit in a second PCB portion. In this case, the electronic circuitry of the eye descriptor unit 1 connecting the eye sensors 12 to the edge computing unit 10 is designed accordingly.

The eye descriptor unit 1, is designed according to the edge computing principles, i.e. the computation of the data occurs where or close to where the input data are generated. The eye sensors 12 generate the eye representation data which are processed directly by the edge computation unit 10 in the eye descriptor unit 1.

The eye descriptor unit 1 (fig. 1) is provided with at least one eye sensor 12 facing the user's left eye and one eye sensor 12 facing the user's right eye and an edge computation unit 10 provided with a machine learning hardware accelerator support configured to receive as input 120 unprocessed user's eye representation data from the eye sensors 12 and to send as output 20 an array of user's eye-descriptors when in use and wherein the edge computation unit 10 is programmed with a customized bare metal code or with Real-Time Operating System RTOS for microcontrollers or with a combination of customized bare metal code and Real-Time Operating System RTOS for microcontrollers.

The eye sensors 12 are configured to face the user's eye once the eye descriptor unit 1 is implemented in the structure of the computer application device 2. In this regard, one preferred solution is arranging the eye sensor 12 in a first U-shaped PCB nose-bridge part 30 and the edge computing unit 10 in a second PCB part 31.

According to an embodiment, compatible with all the possible different embodiments of the present specification, the second PCB part 31 is an extension of the first U-shaped PCB nose-bridge part 30, which is shaped to be integrated into a Wearable U-shaped nose bridge portion compatible with the nose of a wearer when in use.

According to a further embodiment, compatible with all the possible different embodiments of the present specification, the second PCB part 31 is electronically connected to the first U-shaped PCB nose-bridge part 30, but it may be arranged in a different area other than a Wearable U-shaped nose bridge portion compatible with the nose of a wearer when in use.

The output 20, being the array of user's eye descriptor, is provided referred to the extrinsic parameters (rotations about the axes x, y, z of the original coordinate system of the eye descriptor unit 1) of the eye descriptor unit 1.

The output 20, being the array of user's eye descriptor, may include x, y, z-components of the 3D user's gaze directions based on the extrinsic parameters of the eye descriptor unit 1 and according to further possible embodiments, it may include other eye geometry parameters (eyeball center 3D coordinates, pupil center 3D coordinates), and/or pupil size data, and/or blink state, and/or vergence angle.

In eye-tracking systems, accurately estimating the user's eye descriptors, in particular user's gaze direction, relies on understanding the geometry of the user's eye, including estimating parameters such as the corneal curvature, pupil center, and the relative positioning of the eye structures. These geometrical details are relevant to having accurate gaze direction measurements because the eye's anatomy affects the path of light entering the eye and the reflection points observed by tracking cameras. Without eye geometry estimation, gaze estimation algorithms would not perform properly, resulting in reduced accuracy and robustness of eye-tracking pipeline, especially across different users.

With regard to the edge computation unit 10, one preferred solution is that it is a microcontroller programmed with a customized bare metal code or with Real-Time Operating System RTOS for microcontrollers or with a combination of customized bare metal code and Real-Time Operating System RTOS for microcontrollers, in order to achieve very low power consumption.

Bare-metal systems operate without the integration of an operating system, enabling application code to execute directly on the hardware. This configuration allows the application to interact with and access hardware resources without the mediation of an operating system layer.

In contrast, general-purpose operating systems (GPOS), such as Windows, Linux, and MacOS, typically introduce performance overhead due to their kernel operations, which can result in jitter and delays in response times. By facilitating direct hardware access, bare-metal systems enhance the ability to maintain precise timing and deterministic behavior, thereby ensuring minimal latency in system operations.

It may be useful to clarify that a controller is an extremely general term to define a device or system component designed to manage, regulate, or direct the operation of other devices or systems. Controllers execute commands based on inputs they receive, process these commands through programmed logic or algorithms, and produce outputs that adjust or control certain behaviors within a system. They can vary in complexity, from simple analog controllers that respond to electrical signals to complex digital controllers that execute software-based instructions. Controller may be: PLC, Microprocessor, Microcontrollers, FPGA (like for instance ICE40 from Lattice Semiconductors), Motor controller.

Furthermore, it's necessary to distinguish between microprocessor and microcontrollers. According to an article written on 13/6/2024 by Josh Schneider on the IBM website (https://www.ibm.com/think/topics/microcontroller-vs-microprocessor):
*"On a hardware level, microprocessors are based on the "classical" von Neumann architecture. This consists* of a *CPU with both an arithmetic logic unit (ALU) and processor registers (small amounts of fast memory storage for quick data access), a control unit, memory for data and instructions, external memory for mass storage, and I*/*O mechanisms. This methodology uses the same set* of *interconnecting wires (known as a bus) to both transmit instructions and perform operations. Microprocessors cannot perform these actions simultaneously, yet modern devices use various mitigation techniques to avoid data bottlenecks.*
*On the other hand, microcontrollers use the more complex Harvard architecture, which has one dedicated set of data buses and address buses for reading and writing data to memory, and another set to fetch instructions for performing operations. Since the CPU can both read an instruction and access data memory at the same time, the Harvard architecture can perform basic operations faster."*

Regarding the hardware configuration, John Schneider explains and distinguishes:
*"Key components of a microcontroller*
   - *Central processing unit (CPU): Colloquially referred to as the computer's "brain," the CPU is responsible for executing instructions and controlling operations.*
   - *Memory: Microcontrollers contain both volatile memory (RAM), which stores temporary data that may be lost if the system loses power, and non-volatile flash memory (ROM) for storing the microcontroller's programming code.*
   - *Peripherals: Depending on the intended application, a microcontroller may contain various peripheral components, such as I*/*O interfaces, timers, counters, analog-to-digital converters (ADCs) and communication protocols (UART, SPI, 12C).*
   *(...)*
*Key components of a microprocessor*
   *Modern microprocessors combine millions of small transistors, resistors and diodes assembled on a semiconductor material to create the key components of a CPU.*
   - *Arithmetic logic unit (ALU): The main logic unit of the CPU, this component executes logical operations including mathematical calculations and data comparisons.*
   - *Control unit (CU): The CU circuit interprets instructions and initiates their execution, directing the basic operations of the processor.*
   - *Registers: Small, fast memory storage used by a CPU to temporarily hold data and instructions during computational processes.*
   - *Cache memory: Microprocessors and CPUs use cache memory, a high-speed form of memory located close to the CPU, to store frequently accessed data to accelerate performance.*
   - *Processor cores: Individual processing units within microprocessors are known* as *cores. Modern processors frequently incorporate multiple cores (dual-core, quad-core) allowing for parallel processing by enabling the performance of multiple tasks simultaneously.*
   - *I*/*O modules: A microprocessor's I*/*O components are critical for managing the flow* of *data to and from the CPU, including any additional computer peripherals including networking peripherals such as ethernet ports or WiFi units."*

Therefore, a microcontroller (MCU) is a compact, integrated computing device designed for deterministic control of hardware systems. In essence, it is a very specific controller hardware architecture. It operates either without an operating system, known as "bare metal," or with a "Real-Time Operating System (RTOS)", or with a combination of customized bare metal code and Real-Time Operating System RTOS for microcontrollers, providing predictable, time-bound task execution essential for real-time applications and very power efficient, and both of them may be used in the eye descriptor unit 1 of the present invention. Unlike general-purpose processors (like for instance CortexA architecture as Cortex-A53), microcontrollers do not support general-purpose operating systems (e.g., Windows or Linux) due to their focus on efficient, low-level control and minimal latency requirements.

Multiple types of RTOS are available, and they vary based on their target hardware and design goals. RTOS can generally be classified as either lightweight RTOS (for microcontrollers) or more robust RTOS (for processors). Microcontroller RTOS are focused on minimal footprint and low power consumption, handling simpler real-time tasks. Processor RTOS are designed for more complex systems, often with multitasking, advanced peripherals, and networking. RTOS for Microcontrollers: are designed for constrained environments (limited memory, processing power, and power consumption).

Examples may be FreeRTOS, Micrium OS (uC/OS), Zephyr OS, ThreadX (Azure RTOS), Mbed OS.

Furthermore, a defining technical feature of microcontrollers is their inclusion of internal peripherals, such as Analog-to-Digital Converters (ADCs), timers, and communication interfaces (e.g., UART, SPI, and I2C) and GPIOs. These integrated peripherals enable direct, low-latency interaction with external sensors, and actuators, such as cameras, LEDs and communication modules, which is necessary for precise, real-time system control.

Microcontrollers are also optimized for low-power operation, with the capability to achieve sub-millisecond wake-up times from sleep modes. This feature allows a microcontroller-based system to enter low-power states between processing intervals or frames, making it suitable for applications with strict power management requirements.

A microcontroller relies on deterministic interrupting handling systems, which ensures that they can respond rapidly to hardware events, typically within a few microseconds. Microcontrollers are designed to prioritize interruptions with minimal latency, allowing them to address high-priority events in real-time. In general-purpose systems, interrupting latency is often less predictable due to the overhead of multitasking operating systems and other background processes.

The interrupt handling, plus a bare metal or RTOS or both allows for a deterministic system, avoiding most of the problems of synchronization.

The left and right eye sensors 12 may be camera-based sensors but may be different kinds of technologies like lasers able to scan user's eyes to detect position of pupils or micro electromechanical systems like for instance MEMS-based scanning elements.

When the eye sensors 12 are camera-based sensors, they may be monochromatic cameras, DVS cameras for example or other cameras. In that case, the eye representations are eye images where at least the user's pupil is included.

When the eye sensors 12 are camera-based sensors the eye descriptor unit 1 may include optics 18, allowing to control the amount of light that enters the cameras.

When eye sensor 12 are camera-based sensors the eye descriptor unit 1 may include Infrared LED. Infrared (IR) LEDs are components in eye-tracking devices, primarily because they provide a source of invisible light that illuminates the eyes without causing distraction, as IR light lies outside the visible spectrum. This illumination helps to capture clear images of the eyes, even in low-light conditions.

The IR light may also enhance contrast for key eye features, such as the pupil and iris. It also creates a bright reflection, known as the "glint," by reflecting off the retina, which helps distinguish the pupil from other parts of the eye.

In order to widen the possible biomarker detectable by the eye descriptor unit according to the present invention, in every possible embodiment already described, the eye descriptor unit 1 may further comprise an Ambient Light Sensor 14 (ALS), being connected to the edge computation unit 10, to provide further ambient descriptors, namely ambient light descriptors, being included in the output 20 as an array of user's eye descriptors.

In eye-tracking research, ambient light sensors also provide distinct advantages when detecting and interpreting biomarkers related to cognitive states, such as cognitive load, stress, fatigue, and emotional arousal. Eye-based biomarkers-such as pupil dilation, blink rate, and saccadic velocity-are sensitive to external lighting, as pupil size, in particular, responds not only to cognitive load but also to changes in ambient light or adjust the changes due to narcotics. Without an ambient light sensor, variations in room lighting could cause fluctuations in pupil size unrelated to cognitive or emotional states, complicating data interpretation. By accounting for ambient light and adjusting the IR illumination accordingly, the sensor helps isolate true, cognitive-related pupil dilation from light-induced changes.

This capability is crucial for applications in psychology, neuroscience, and even commercial settings where eye-tracking is used to assess the cognitive load, emotional engagement, or decision-making under various conditions. For example, accurate measurements of pupil dilation can indicate cognitive workload in pilots, drivers, or students during high-stakes tasks. Similarly, eye-tracking insights into stress and fatigue levels can be harnessed in workplace safety and mental health monitoring.

Furthermore, in order to widen the possible biomarker detectable by the eye descriptor unit according to the present invention possibly connected to the user's movements, in every possible embodiment already described, the eye descriptor unit 1 may further comprise an Inertial Movement Unit 16 (IMU), being connected to the edge computation unit 10, to provide further user's descriptors as for example head pose descriptors, being included in the output 20 as an array of eye descriptors.

The inclusion of an IMU in eye-tracking devices provides additional advantages for detecting sophisticated oculomotor biomarkers, such as smooth pursuit, vestibulo-ocular reflex (VOR), and other eye movement behaviors linked to cognitive and neurological health and micro muscular movements. Smooth pursuit, the ability to track a moving object with coordinated eye movements, is an important biomarker used in studies of attention, focus, and neurological function. By combining IMU data with eye-tracking data, the system can more accurately distinguish between smooth pursuit and head movement, especially in natural, mobile environments where both are likely to occur simultaneously.

For detecting and studying vestibulo-ocular reflex (VOR) and vestibulo-ocular locking, which are critical for gaze stability during head movement, the IMU provides head motion data that allows the eye-tracker to discern eye movements compensating for head rotation. This separation is valuable for research in fields like neuro-ophthalmology and vestibular therapy, where VOR function is essential for balance and spatial orientation. Furthermore, IMU-enhanced eye-tracking enables detailed studies of other eye movement patterns, such as saccades, fixations, and microsaccades, in conjunction with head movements, adding depth to biomarker analyses related to cognitive workload, fatigue, and attentional control. By isolating and analyzing these nuanced eye-head interactions, the IMU improves the reliability and interpretability of biomarker data, expanding the utility of eye-tracking technology in clinical diagnostics, cognitive assessment, and neurology.

In each embodiment disclosed in the present specification the edge computation unit 10, the memory and the data interface may be connected at least indirectly to an energy accumulator by circuitry.

In each embodiment disclosed in the present specification the output 20, being an array of user's eye descriptors, may be a text-based file.

In each embodiment disclosed in the present specification, the eye descriptor unit 1 may have a maximum power consumption of 200 mW.

In each embodiment disclosed in the present specification, the external interface protocol provided by the eye descriptor unit 1 is configured to exchange data in a master-slave configuration, where the master is the computer application device 2 which initiates and controls the communication using separate lines for clock signaling and data, where the eye descriptor unit 1 is the slave, and transmits this information serialized. This configuration allows to achieve several advantages: high compatibility with processors of the computer application device 2, low Pin Count, resistance to EMC, low power consumption, low overhead, and low baud rate compared to higher complexity protocols including more overhead.

The present invention furthermore relates to a method for detecting user's eye descriptors using an eye descriptor unit 1 configured to receive as input 120 unprocessed user's eye representation data from eye sensors 12, to process them by a machine learning hardware accelerator support and to send as output 20, an array of user's eye-descriptors, when in use connected to a computer application device 2 and wherein the edge computation unit 10 is a microcontroller, and wherein at least one eye sensor 12 is configured facing the user's left eye and one eye sensor 12 is configured facing the user's right eye and both eye sensors 12 are arranged in a first U-shaped PCB nose bridge part 30, while the edge computing unit 10 is arranged in a second PCB part 31 and wherein the edge computing unit 10 is a microcontroller programmed with a customized bare metal code or with Real-Time Operating System RTOS for microcontrollers or with a combination of customized bare metal code and Real-Time Operating System RTOS for microcontrollers.

**In** a further embodiment compatible with all possible variations described in the present specification, the invented method for detecting user's eye descriptors using an eye descriptor unit 1, once the eye descriptor unit 1 according to any embodiments of the present invention is connected to a computer application device 2 worn by a user, comprises the following steps:
- Receive as input (120) at least two user's eye representations data at two different time instances from the eye sensors
- Estimate the user's eye geometry based on the two user's eye representations
- Send as output (20) the array of eye descriptors of the user

Furthermore, the method may include the following steps:
- Sending, by the edge computation unit 10, as output 20, the array of eye descriptors of the user to the computer application device 2.

**In** a further embodiment compatible with all possible variations described in the present specification, the invented method may use the edge computation unit 10 as being configured to be in a sleep state after it has processed the output 20 eye descriptor array and until it receives a wake-up request from the computer application device 2.

Moreover, the method may also implement that the output 20, as eye descriptor array, includes the x, y and z-components of the 3D gaze direction of the user, based on the eye descriptor unit 1 extrinsic parameters and/or further eye geometry parameters, and/or pupil size data, and/or blink state, and/or vergence angle, and/or further user's descriptors, and/or further ambient descriptors.

The method according to all possible variants described in the present specification, may use the edge computation unit 10 being a microcontroller.

The present invention furthermore relates to the computer application device 2 connected to the eye descriptor unit 1 according to all possible embodiments described in the present specification and/or implementing the method already described in all its possible variants.

The disclosed invention presents a highly cost-effective and power-efficient eye descriptor unit 1 also called eye/gaze-tracking module, addressing critical challenges in wearable and portable device applications. By leveraging optimized hardware integration, this technology significantly reduces manufacturing costs and operational power requirements without compromising accuracy or reliability. These advancements enable broader accessibility of eye-tracking capabilities across consumer electronics, medical devices, and assistive technologies, promoting widespread adoption while extending device battery life and minimizing thermal output. The inventive eye descriptor unit offers a competitive edge by fulfilling industry demands for low-cost, energy-efficient solutions, representing a substantial improvement over existing systems in the field.

## Claims

1. An eye descriptor unit (1) for estimating a user's gaze once the eye descriptor unit (1) is connected to a computer application device (2) worn by the user when in use, said eye descriptor unit (1) comprising a first U-shaped PCB nose bridge part (30) which is shaped for being integrated into a wearable U-shaped nose bridge portion compatible with the nose of the user, said first U-shaped PCB nose bridge part (30) including at least one eye sensor (12) facing the user's left eye and one eye sensor (12) facing the user's right eye, said eye descriptor unit (1) further comprising a second PCB part (31) including an edge computation unit (10) electronically connected to the eye sensors (12) by an electronic circuitry, said edge computation unit (10) provided with a machine learning hardware accelerator support and configured to receive as input (120) unprocessed user's eye representation data from the eye sensors (12) and to send as output (20) an array of user's eye-descriptors when in use and wherein the edge computation unit (10) is a microcontroller programmed with a customized bare metal code or with Real-Time Operating System, RTOS, for microcontrollers or with a combination of customized bare metal code and Real-Time Operating System, RTOS, for microcontrollers.

2. The eye descriptor unit (1) according to claim 1, wherein the edge computation unit (10) is configured to:
- Receive as input (120) at least two user's eye representation data at two different time instances from the eye sensors;
- Estimate the user's eye geometry based on the input (120);
- Send as output (20) the array of eye descriptors of the user.

3. The eye descriptor unit (1) according to claims 1 or 2, wherein the second PCB part (31) including the edge computation unit (10) is an extension of the first U-shaped PCB nose bridge part (30) and it is configured for being integrated into the wearable U-shaped nose bridge portion compatible with the nose of the user when in use.

4. The eye descriptor unit (1), according to any of the preceding claims, wherein the output (20) is computed by using a neural network running in the edge computation unit (10) when in use.

5. The eye descriptor unit (1), according to any of the preceding claims, wherein the output (20) includes x, y and z-components of the 3D gaze direction of the user, based on the eye descriptor unit (1) extrinsic parameters and/or further eye geometry parameters, and/or pupil size data, and/or blink state, and/or vergence angle.

6. The eye descriptor unit (1), according to any of the preceding claims, wherein the eye descriptor unit (1) maximum power consumption is 200 mW.

7. The eye descriptor unit (1), according to any of the preceding claims, wherein the edge computation unit (10) is configured to be in a sleep state after it has processed the output (20) and until it receives a wake-up request from the computer application device (2).

8. The eye descriptor unit (1), according to any of the preceding claims, wherein the eye sensors (12) are cameras.

9. The eye descriptor unit (1), according to claim 8, further comprises infrared LED illuminators.

10. The eye descriptor unit (1), according to any of the preceding claims, including ambient light sensors, ALS, and/or an Inertial movement unit, IMU.

11. The eye descriptor unit (1), according to any of the preceding claims, wherein an external interface protocol provided by the eye descriptor unit (1) is configured to exchange data in a master-slave configuration, where the master being the computer application device (2), initiating and controlling the communication using separate lines for clock signaling and data, and the eye descriptor unit (1) being the slave, transmitting information serialized.

12. A method for detecting user's eye descriptors using an eye descriptor unit (1) configured to receive as input (120) unprocessed user's eye representation data from eye sensors (12), to process them by a machine learning hardware accelerator support and to send as output (20) an array of user's eye-descriptors when in use being connected to a computer application device (2), wherein at least one of the eye sensors (12) is configured to face the user's left eye and one of the eye sensors (12) is configured to face the user's right eye and both eye sensors (12) are arranged in a first U-shaped PCB nose bridge part (30) of the eye descriptor unit (1) while an edge computation unit (10) is arranged in a second PCB part (31) of the eye descriptor unit (1), and wherein the edge computation unit (10) provided with the machine learning hardware accelerator support is a microcontroller programmed with a customized bare metal code or with Real-Time Operating System, RTOS, for microcontrollers or with a combination of customized bare metal code and Real-Time Operating System, RTOS, for microcontrollers.

13. The method according to claim 12, wherein the eye descriptor unit (1) is configured to:
- Receive as input (120) at least two user's eye representation data at two different time instances from the eye sensors (12);
- Estimate the user's eye geometry based on the input (120);
- Send as output (20) the array of eye descriptors of the user when in use.

14. The method according to claim 12 or 13, wherein the edge computation unit (10) is configured to be in a sleep state after it has processed the output (20) and until it receives a wake-up request from the computer application device (2).

15. The method according to any of the claims 12 to 14, wherein the output (20) includes the user gaze vector and/or further user's descriptors as head pose descriptors, and/or further ambient descriptors as ambient light descriptors.

16. A computer application device (2) including the eye descriptor unit (1) according to any of the claims 1 to 11.

## Patentansprüche

1. Augendeskriptoreinheit (1) zum Schätzen der Blickrichtung eines Benutzers, sobald die Augendeskriptoreinheit (1) mit einem im Gebrauch von dem Benutzer getragenen Computeranwendungsgerät (2) verbunden ist, wobei die Augendeskriptoreinheit (1) ein erstes U-förmiges PCB-Nasenbrückenteil (30) umfasst, das zur Integration in einen tragbaren U-förmigen Nasenbrückenabschnitt ausgeformt ist, der mit der Nase des Benutzers kompatibel ist, wobei das erste U-förmige PCB-Nasenbrückenteil (30) mindestens einen dem linken Auge des Benutzers zugewandten Augensensor (12) und einen dem rechten Auge des Benutzers zugewandten Augensensor (12) aufweist, wobei die Augendeskriptoreinheit (1) ferner ein zweites PCB-Teil (31) umfasst, das eine über eine elektronische Schaltung elektronisch mit den Augensensoren (12) verbundene Edge-Recheneinheit (10) aufweist, wobei die Edge-Recheneinheit (10) mit einer Hardwarebeschleunigerunterstützung für maschinelles Lernen versehen und dazu konfiguriert ist, im Gebrauch als Eingabe (120) unverarbeitete Augendarstellungsdaten des Benutzers von den Augensensoren (12) zu empfangen und als Ausgabe (20) ein Array von Augendeskriptoren des Benutzers zu senden, und wobei die Edge-Recheneinheit (10) ein Mikrocontroller ist, der mit einem angepassten Bare-Metal-Code oder mit einem Echtzeitbetriebssystem, RTOS, für Mikrocontroller oder mit einer Kombination aus einem angepassten Bare-Metal-Code und einem Echtzeitbetriebssystem, RTOS, für Mikrocontroller programmiert ist.

2. Augendeskriptoreinheit (1) nach Anspruch 1, wobei die Edge-Recheneinheit (10) zu Folgendem konfiguriert ist:
- Empfangen, als Eingabe (120), von mindestens zwei Augendarstellungsdaten des Benutzers zu zwei unterschiedlichen Zeitpunkten von den Augensensoren (12);
- Schätzen der Augengeometrie des Benutzers auf Grundlage der Eingabe (120);
- Senden, als Ausgabe (20), des Arrays von Augendeskriptoren des Benutzers.

3. Augendeskriptoreinheit (1) nach Anspruch 1 oder 2, wobei das die Edge-Recheneinheit (10) aufweisende zweite PCB-Teil (31) eine Erweiterung des ersten U-förmigen PCB-Nasenbrückenteils (30) ist und dazu konfiguriert ist, im Gebrauch in den tragbaren U-förmigen Nasenbrückenabschnitt integriert zu werden, der mit der Nase des Benutzers kompatibel ist.

4. Augendeskriptoreinheit (1) nach einem der vorhergehenden Ansprüche, wobei die Ausgabe (20) unter Verwendung eines im Gebrauch in der Edge-Recheneinheit (10) ausgeführten neuronalen Netzes berechnet wird.

5. Augendeskriptoreinheit (1) nach einem der vorhergehenden Ansprüche, wobei die Ausgabe (20) auf Grundlage der extrinsischen Parameter der Augendeskriptoreinheit (1) die x-, y- und z-Komponenten der 3D-Blickrichtung des Benutzers und/oder weitere Augengeometrieparameter und/oder Pupillengrößendaten und/oder einen Blinzelzustand und/oder einen Vergenzwinkel umfasst.

6. Augendeskriptoreinheit (1) nach einem der vorhergehenden Ansprüche, wobei die maximale Leistungsaufnahme der Augendeskriptoreinheit (1) 200 mW beträgt.

7. Augendeskriptoreinheit (1) nach einem der vorhergehenden Ansprüche, wobei die Edge-Recheneinheit (10) dazu konfiguriert ist, sich, nachdem sie die Ausgabe (20) verarbeitet hat, und bis sie eine Aufweckanforderung von dem Computeranwendungsgerät (2) empfängt, in einem Schlafzustand zu befinden.

8. Augendeskriptoreinheit (1) nach einem der vorhergehenden Ansprüche, wobei die Augensensoren (12) Kameras sind.

9. Augendeskriptoreinheit (1) nach Anspruch 8, ferner umfassend Infrarot-LED-Beleuchtungseinrichtungen.

10. Augendeskriptoreinheit (1) nach einem der vorhergehenden Ansprüche, umfassend Umgebungslichtsensoren, ALS, und/oder eine Inertialbewegungseinheit, IMU.

11. Augendeskriptoreinheit (1) nach einem der vorhergehenden Ansprüche, wobei ein von der Augendeskriptoreinheit (1) bereitgestelltes externes Schnittstellenprotokoll dazu konfiguriert ist, Daten in einer Master-Slave-Konfiguration auszutauschen, wobei der Master das Computeranwendungsgerät (2) ist, das die Kommunikation unter Verwendung getrennter Leitungen für Taktsignale und Daten initiiert und steuert, und wobei die Augendeskriptoreinheit (1) der Slave ist, der Informationen serialisiert überträgt.

12. Verfahren zum Erfassen von Augendeskriptoren eines Benutzers unter Verwendung einer Augendeskriptoreinheit (1), die dazu konfiguriert ist, als Eingabe (120) unverarbeitete Augendarstellungsdaten des Benutzers von Augensensoren (12) zu empfangen, diese mittels einer Hardwarebeschleunigerunterstützung für maschinelles Lernen zu verarbeiten und im Gebrauch, während sie mit einem Computeranwendungsgerät (2) verbunden ist, als Ausgabe (20) ein Array von Augendeskriptoren des Benutzers zu senden, wobei mindestens einer der Augensensoren (12) dazu konfiguriert ist, dem linken Auge des Benutzers zugewandt zu sein, und einer der Augensensoren (12) dazu konfiguriert ist, dem rechten Auge des Benutzers zugewandt zu sein, und beide Augensensoren (12) in einem ersten U-förmigen PCB-Nasenbrückenteil (30) der Augendeskriptoreinheit (1) angeordnet sind, während eine Edge-Recheneinheit (10) in einem zweiten PCB-Teil (31) der Augendeskriptoreinheit (1) angeordnet ist, und wobei die mit der Hardwarebeschleunigerunterstützung für maschinelles Lernen versehene Edge-Recheneinheit (10) ein Mikrocontroller ist, der mit einem angepassten Bare-Metal-Code oder mit einem Echtzeitbetriebssystem, RTOS, für Mikrocontroller oder mit einer Kombination aus einem angepassten Bare-Metal-Code und einem Echtzeitbetriebssystem, RTOS, für Mikrocontroller programmiert ist.

13. Verfahren nach Anspruch 12, wobei die Augendeskriptoreinheit (1) zu Folgendem konfiguriert ist:
- Empfangen, als Eingabe (120), von mindestens zwei Augendarstellungsdaten des Benutzers zu zwei unterschiedlichen Zeitpunkten von den Augensensoren (12);
- Schätzen der Augengeometrie des Benutzers auf Grundlage der Eingabe (120);
- Senden, als Ausgabe (20), des Arrays von Augendeskriptoren des Benutzers im Gebrauch.

14. Verfahren nach Anspruch 12 oder 13, wobei die Edge-Recheneinheit (10) dazu konfiguriert ist, sich, nachdem sie die Ausgabe (20) verarbeitet hat, und bis sie eine Aufweckanforderung von dem Computeranwendungsgerät (2) empfängt, in einem Schlafzustand zu befinden.

15. Verfahren nach einem der Ansprüche 12 bis 14, wobei die Ausgabe (20) den Blickvektor des Benutzers und/oder weitere Benutzerdeskriptoren in Form von Kopfhaltungsdeskriptoren und/oder weitere Umgebungsdeskriptoren in Form von Umgebungslichtdeskriptoren umfasst.

16. Computeranwendungsgerät (2), umfassend die Augendeskriptoreinheit (1) nach einem der Ansprüche 1 bis 11.

## Revendications

1. Unité de descripteurs oculaires (1) destinée à estimer le regard d'un utilisateur une fois que l'unité de descripteurs oculaires (1) est connectée à un dispositif d'application informatique (2) porté par l'utilisateur lors de l'utilisation, ladite unité de descripteurs oculaires (1) comprenant une première partie de pont nasal de PCB en forme de U (30), qui est conformée pour être intégrée dans une portion de pont nasal portable en forme de U compatible avec le nez de l'utilisateur, ladite première partie de pont nasal de PCB en forme de U (30) comprenant au moins un capteur oculaire (12) faisant face à l'œil gauche de l'utilisateur et un capteur oculaire (12) faisant face à l'œil droit de l'utilisateur, ladite unité de descripteurs oculaires (1) comprenant en outre une seconde partie de PCB (31) comprenant une unité d'edge computing (10) connectée électroniquement aux capteurs oculaires (12) par un circuit électronique, ladite unité d'edge computing (10) étant pourvue d'un support d'accélérateur matériel d'apprentissage automatique et configurée pour recevoir, en tant qu'entrée (120), des données non traitées de représentation oculaire de l'utilisateur provenant des capteurs oculaires (12) et pour envoyer, en tant que sortie (20), un tableau de descripteurs oculaires de l'utilisateur lors de l'utilisation, et dans laquelle l'unité d'edge computing (10) est un microcontrôleur programmé avec un code bare metal personnalisé ou avec un système d'exploitation en temps réel, RTOS, pour microcontrôleurs, ou avec une combinaison d'un code bare metal personnalisé et d'un système d'exploitation en temps réel, RTOS, pour microcontrôleurs.

2. Unité de descripteurs oculaires (1) selon la revendication 1, dans laquelle l'unité d'edge computing (10) est configurée pour :
- Recevoir, en tant qu'entrée (120), au moins deux données de représentation oculaire de l'utilisateur à deux instants différents provenant des capteurs oculaires (12) ;
- Estimer la géométrie oculaire de l'utilisateur sur la base de l'entrée (120) ;
- Envoyer, en tant que sortie (20), le tableau de descripteurs oculaires de l'utilisateur.

3. Unité de descripteurs oculaires (1) selon la revendication 1 ou 2, dans laquelle la seconde partie de PCB (31) comprenant l'unité d'edge computing (10) constitue un prolongement de la première partie de pont nasal de PCB en forme de U (30) et est configurée pour être intégrée, lors de l'utilisation, dans la portion de pont nasal portable en forme de U compatible avec le nez de l'utilisateur.

4. Unité de descripteurs oculaires (1) selon l'une quelconque des revendications précédentes, dans laquelle la sortie (20) est calculée à l'aide d'un réseau neuronal s'exécutant dans l'unité d'edge computing (10) lors de l'utilisation.

5. Unité de descripteurs oculaires (1) selon l'une quelconque des revendications précédentes, dans laquelle la sortie (20) comprend, sur la base des paramètres extrinsèques de l'unité de descripteurs oculaires (1), les composantes x, y et z de la direction tridimensionnelle du regard de l'utilisateur et/ou d'autres paramètres de géométrie oculaire, et/ou des données de taille de pupille, et/ou un état de clignement, et/ou un angle de vergence.

6. Unité de descripteurs oculaires (1) selon l'une quelconque des revendications précédentes, dans laquelle la consommation électrique maximale de l'unité de descripteurs oculaires (1) est de 200 mW.

7. Unité de descripteurs oculaires (1) selon l'une quelconque des revendications précédentes, dans laquelle l'unité d'edge computing (10) est configurée pour se trouver dans un état de veille après avoir traité la sortie (20) et jusqu'à ce qu'elle reçoive une demande de réveil provenant du dispositif d'application informatique (2).

8. Unité de descripteurs oculaires (1) selon l'une quelconque des revendications précédentes, dans laquelle les capteurs oculaires (12) sont des caméras.

9. Unité de descripteurs oculaires (1) selon la revendication 8, comprenant en outre des dispositifs d'éclairage à LED infrarouges.

10. Unité de descripteurs oculaires (1) selon l'une quelconque des revendications précédentes, comprenant des capteurs de lumière ambiante, ALS, et/ou une unité de mouvement inertiel, IMU.

11. Unité de descripteurs oculaires (1) selon l'une quelconque des revendications précédentes, dans laquelle un protocole d'interface externe fourni par l'unité de descripteurs oculaires (1) est configuré pour échanger des données dans une configuration maître-esclave, le maître étant le dispositif d'application informatique (2), qui initie et commande la communication en utilisant des lignes distinctes pour le signal d'horloge et les données, et l'unité de descripteurs oculaires (1) étant l'esclave, qui transmet des informations sous forme sérialisée.

12. Procédé de détection de descripteurs oculaires d'un utilisateur au moyen d'une unité de descripteurs oculaires (1) configurée pour recevoir, en tant qu'entrée (120), des données non traitées de représentation oculaire de l'utilisateur provenant de capteurs oculaires (12), pour les traiter au moyen d'un support d'accélérateur matériel d'apprentissage automatique et pour envoyer, en tant que sortie (20), un tableau de descripteurs oculaires de l'utilisateur lors de l'utilisation en étant connectée à un dispositif d'application informatique (2), dans lequel au moins l'un des capteurs oculaires (12) est configuré pour faire face à l'œil gauche de l'utilisateur et l'un des capteurs oculaires (12) est configuré pour faire face à l'œil droit de l'utilisateur, les deux capteurs oculaires (12) étant disposés dans une première partie de pont nasal de PCB en forme de U (30) de l'unité de descripteurs oculaires (1), tandis qu'une unité d'edge computing (10) est disposée dans une seconde partie de PCB (31) de l'unité de descripteurs oculaires (1), et dans lequel l'unité d'edge computing (10), pourvue du support d'accélérateur matériel d'apprentissage automatique, est un microcontrôleur programmé avec un code bare metal personnalisé ou avec un système d'exploitation en temps réel, RTOS, pour microcontrôleurs, ou avec une combinaison d'un code bare metal personnalisé et d'un système d'exploitation en temps réel, RTOS, pour microcontrôleurs.

13. Procédé selon la revendication 12, dans lequel l'unité de descripteurs oculaires (1) est configurée pour :
- Recevoir, en tant qu'entrée (120), au moins deux données de représentation oculaire de l'utilisateur à deux instants différents provenant des capteurs oculaires (12) ;
- Estimer la géométrie oculaire de l'utilisateur sur la base de l'entrée (120) ;
- Envoyer, en tant que sortie (20), le tableau de descripteurs oculaires de l'utilisateur lors de l'utilisation.

14. Procédé selon la revendication 12 ou 13, dans lequel l'unité d'edge computing (10) est configurée pour se trouver dans un état de veille après avoir traité la sortie (20) et jusqu'à ce qu'elle reçoive une demande de réveil provenant du dispositif d'application informatique (2).

15. Procédé selon l'une quelconque des revendications 12 à 14, dans lequel la sortie (20) comprend le vecteur de regard de l'utilisateur et/ou d'autres descripteurs de l'utilisateur sous la forme de descripteurs de pose de la tête, et/ou d'autres descripteurs ambiants sous la forme de descripteurs de lumière ambiante.

16. Dispositif d'application informatique (2) comprenant l'unité de descripteurs oculaires (1) selon l'une quelconque des revendications 1 à 11.
